(19) Europäisches Patentamt
European Patent Office
Office européen des brevets

(11) EP 4 786 468 A1

(12) **EUROPEAN PATENT APPLICATION**
published in accordance with Art. 153(4) EPC

(43) Date of publication:
**05.08.2026 Bulletin 2026/32**

(21) Application number: **24870953.7**

(22) Date of filing: **27.09.2024**

(51) International Patent Classification (IPC):
***C07D 417/04*** *(2006.01)* ***A61K 31/545*** *(2006.01)*
***A61K 31/5415*** *(2006.01)* ***A61P 35/00*** *(2006.01)*
***A61P 25/28*** *(2006.01)*

(52) Cooperative Patent Classification (CPC):
**A61K 31/5415; A61K 31/545; A61P 25/28; A61P 35/00; C07D 417/04**

(86) International application number:
**PCT/CN2024/121715**

(87) International publication number:
**WO 2025/067416 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311277270**

(71) Applicant: **Suzhou Genhouse Bio Co., Ltd.**
**Suzhou,Jiangsu 215123 (CN)**

(72) Inventors:
- **ZHANG, Guiping**
  **Suzhou, Jiangsu 215123 (CN)**
- **ZUO, Gaolei**
  **Suzhou, Jiangsu 215123 (CN)**
- **CUI, Jiuen**
  **Suzhou, Jiangsu 215123 (CN)**
- **GOU, Donghui**
  **Suzhou, Jiangsu 215123 (CN)**
- **LI, Jinjing**
  **Suzhou, Jiangsu 215123 (CN)**

(74) Representative: **Ter Meer Steinmeister & Partner Patentanwälte mbB**
**Nymphenburger Straße 4**
**80335 München (DE)**

(54) **CRYSTALLINE FORM OF HDAC6 INHIBITOR AND PREPARATION METHOD THEREFOR**

(57) A crystalline form of a compound represented by formula (I) and a preparation method thereof. The crystal form can be used for preventing and/or treating HDAC6-mediated or -dependent diseases or conditions.

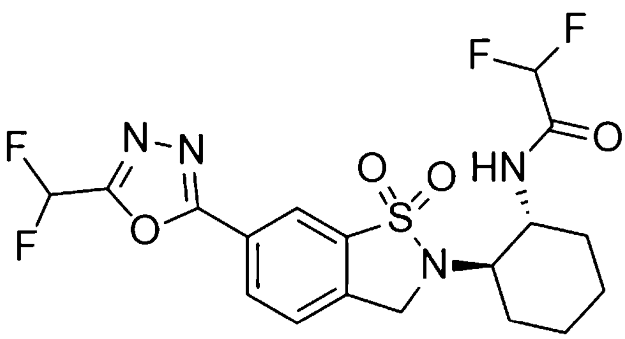

(I)

EP 4 786 468 A1

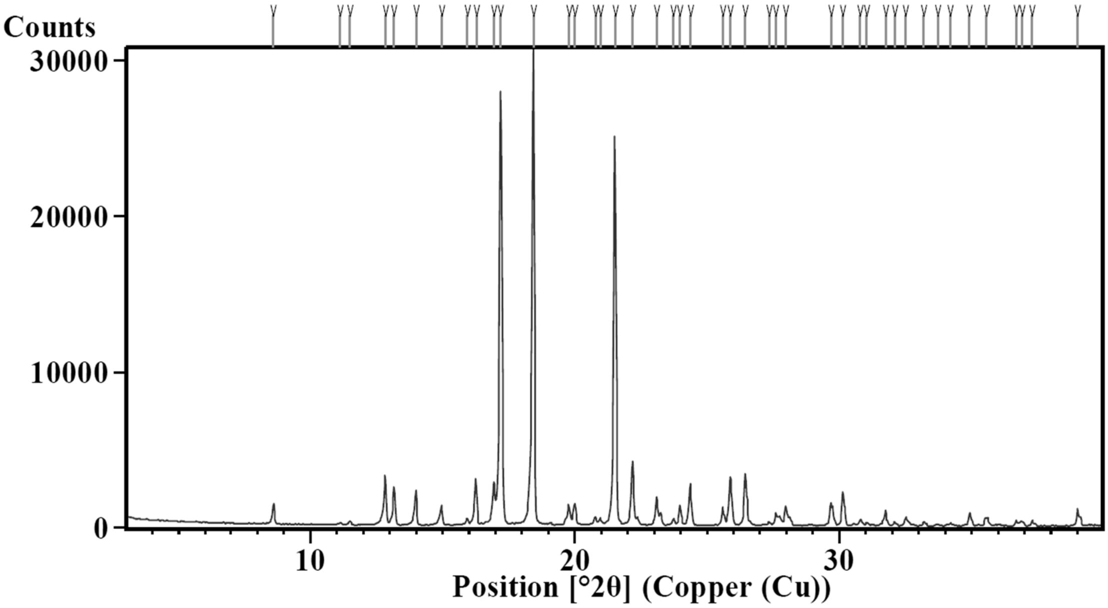


Figure 1

## Description

## CROSS REFERENCE TO RELATED APPLICATION

The present disclosure claims priority to Chinese Patent Application No. 202311277270.2, filed with the China National Intellectual Property Administration on September 28, 2023, entitled "CRYSTALLINE FORM OF AN HDAC6 INHIBITOR AND PREPARATION METHOD THEREOF", the entire contents of which are incorporated herein by reference.

## TECHNICAL FIELD

This disclosure relates to a crystalline form of an HDAC6 inhibitor and preparation method thereof. Specifically, it provides a crystalline form of the compound represented by formula (I) and preparation method thereof.

## BACKGROUND

Histone deacetylases (HDACs) can catalyze the deacetylation of histones or other proteins and play important roles in various biological processes, primarily through transcriptional repression. HDAC6 primarily catalyzes the deacetylation of non-histone substrates, such as α-tubulin and Hsp90. HDAC6 is involved in the pathological processes of various diseases, including cancer, neurological disorders, infections, cardiovascular diseases, immune-related diseases, and inflammatory diseases.

In the pharmaceutical field, it is known that the crystalline form of a compound affects its solubility, stability, compressibility, etc., thereby influencing the safety and efficacy of the drug. Therefore, discovering new crystalline forms of a drug can provide various advantages.

## SUMMARY

The present disclosure provides a crystalline form of the compound represented by formula (I), a preparation method thereof, and applications thereof. The crystalline form of the present disclosure has excellent stability and high crystallinity, which is of significant value for drug optimization and development.

Specifically, the present disclosure provides a crystalline form I of the compound represented by formula (I),

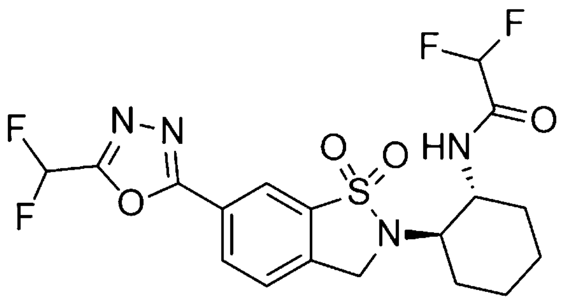

(I),

The X-ray powder diffraction pattern, expressed in terms of diffraction angle 2θ, exhibits characteristic peaks at 17.0931 ± 0.2°, 18.3337 ± 0.2°, 21.4220 ± 0.2°, 22.0595 ± 0.2°, and 26.3492 ± 0.2°. Preferably, it further comprises characteristic peaks at 12.7282 ± 0.2°, 16.1708 ± 0.2°, 16.8430 ± 0.2°, 17.0931 ± 0.2°, 18.3337 ± 0.2°, 21.4220 ± 0.2°, 22.0595 ± 0.2°, 24.2450 ± 0.2°, 25.7696 ± 0.2°, and 26.3492 ± 0.2°. More preferably, it further exhibits characteristic peaks at 12.7282 ± 0.2°, 13.0778 ± 0.2°, 13.8888 ± 0.2°, 16.1708 ± 0.2°, 16.8430 ± 0.2°, 17.0931 ± 0.2°, 18.3337 ± 0.2°, 19.8849 ± 0.2°, 21.4220 ± 0.2°, 22.0595 ± 0.2°, 23.0060 ± 0.2°, 24.2450 ± 0.2°, 25.7696 ± 0.2°, 26.3492 ± 0.2°, and 29.5926 ± 0.2°. Most preferably, it further exhibits characteristic peaks at 12.7282 ± 0.2°, 13.0778 ± 0.2°, 13.8888 ± 0.2°, 14.8709 ± 0.2°, 16.1708 ± 0.2°, 16.8430 ± 0.2°, 17.0931 ± 0.2°, 18.3337 ± 0.2°, 19.6726 ± 0.2°, 19.8849 ± 0.2°, 21.4220 ± 0.2°, 22.0595 ± 0.2°, 23.0060 ± 0.2°, 24.2450 ± 0.2°, 25.4835 ± 0.2°, 25.7696 ± 0.2°, 26.3492 ± 0.2°, 27.5202 ± 0.2°, 29.5926 ± 0.2°, and 30.0442 ± 0.2°.

Furthermore, it exhibits characteristic peaks at 8.5090 ± 0.2°, 11.4131 ± 0.2°, 12.7282 ± 0.2°, 13.0778 ± 0.2°, 13.8888 ± 0.2°, 14.8709 ± 0.2°, 15.8155 ± 0.2°, 16.1708 ± 0.2°, 16.8430 ± 0.2°, 17.0931 ± 0.2°, 18.3337 ± 0.2°, 18.9954 ± 0.2°, 19.6726 ± 0.2°, 19.8849 ± 0.2°, 20.6845 ± 0.2°, 20.8631 ± 0.2°, 21.4220 ± 0.2°, 22.0595 ± 0.2°, 22.2748 ± 0.2°, 23.0060 ± 0.2°, 23.6149 ± 0.2°, 23.8802 ± 0.2°, 24.2450 ± 0.2°, 25.4835 ± 0.2°, 25.7696 ± 0.2°, 26.3492 ± 0.2°, 27.2456 ± 0.2°, 27.5202 ± 0.2°, 27.8593 ± 0.2°, 28.0475 ± 0.2°, 29.5926 ± 0.2°, 30.0442 ± 0.2°,

30.7152 ± 0.2°, 30.9672 ± 0.2°, 31.6849 ± 0.2°, 31.9795 ± 0.2°, 32.4073 ± 0.2°, 33.1395 ± 0.2°, 34.0516 ± 0.2°, 34.8350 ± 0.2°, 35.4536 ± 0.2°, 35.9251 ± 0.2°, 36.5791 ± 0.2°, 37.2047 ± 0.2°, and 38.9204 ± 0.2°.

Furthermore, the X-ray powder diffraction pattern expressed in terms of diffraction angle 2θ is shown in Figure 1.

Furthermore, the TGA curve of crystalline form I is shown in Figure 2.

Furthermore, the DSC curve of crystalline form I is shown in Figure 3.

Beneficial Effects

The crystalline form I of the compound represented by formula (I) provided in the present disclosure exhibits advantages in physicochemical properties, formulation processability, and bioavailability. For example, it demonstrates superiority in at least one aspect among melting point, solubility, stability, and bioavailability. The crystalline form I of the compound represented by formula (I) provided herein possesses favorable crystallinity and process operability, along with excellent stability, thereby offering a superior option for the development of HDAC6 inhibitor drugs and holding significant importance.

## BRIEF DESCRIPTION OF THE DRAWINGS

Figure 1 is an X-ray powder diffraction (XRPD) pattern of crystalline form I of the compound represented by formula (I).

Figure 2 is a thermogravimetric analysis (TGA) curve of crystalline form I of the compound represented by formula (I).

Figure 3 is a differential scanning calorimetry (DSC) curve of crystalline form I of the compound represented by formula (I).

Figure 4 is an XRPD pattern comparison of crystalline form I of the compound represented by formula (I) before and after storage for 1 month under open-dish conditions at 40 °C and 75% relative humidity.

## DETAILED DESCRIPTION

To make the technical solutions and beneficial effects of the present disclosure more clearly understood, detailed description is provided below by way of specific examples. Among them, the drawings are not necessarily drawn to scale, and local features may be enlarged or reduced to more clearly show the details of the local features; unless otherwise defined, the technical and scientific terms used herein have the same meanings as those in the technical field to which the present disclosure belongs.

The present disclosure provides a crystalline form I of the compound represented by formula (I),

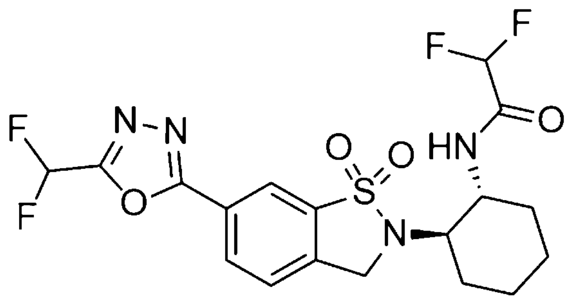

(I),

The X-ray powder diffraction pattern, expressed in terms of diffraction angle 2θ, exhibits characteristic peaks at 17.0931 ± 0.2°, 18.3337 ± 0.2°, 21.4220 ± 0.2°, 22.0595 ± 0.2°, and 26.3492 ± 0.2°. Preferably, it further exhibits characteristic peaks at 12.7282 ± 0.2°, 16.1708 ± 0.2°, 16.8430 ± 0.2°, 17.0931 ± 0.2°, 18.3337 ± 0.2°, 21.4220 ± 0.2°, 22.0595 ± 0.2°, 24.2450 ± 0.2°, 25.7696 ± 0.2°, and 26.3492 ± 0.2°. More preferably, it further exhibits characteristic peaks at 12.7282 ± 0.2°, 13.0778 ± 0.2°, 13.8888 ± 0.2°, 16.1708 ± 0.2°, 16.8430 ± 0.2°, 17.0931 ± 0.2°, 18.3337 ± 0.2°, 19.8849 ± 0.2°, 21.4220 ± 0.2°, 22.0595 ± 0.2°, 23.0060 ± 0.2°, 24.2450 ± 0.2°, 25.7696 ± 0.2°, 26.3492 ± 0.2°, and 29.5926 ± 0.2°. Most preferably, it further exhibits characteristic peaks at 12.7282 ± 0.2°, 13.0778 ± 0.2°, 13.8888 ± 0.2°, 14.8709 ± 0.2°, 16.1708 ± 0.2°, 16.8430 ± 0.2°, 17.0931 ± 0.2°, 18.3337 ± 0.2°, 19.6726 ± 0.2°, 19.8849 ± 0.2°, 21.4220 ± 0.2°, 22.0595 ± 0.2°, 23.0060 ± 0.2°, 24.2450 ± 0.2°, 25.4835 ± 0.2°, 25.7696 ± 0.2°, 26.3492 ± 0.2°, 27.5202 ± 0.2°, 29.5926 ± 0.2°, and 30.0442 ± 0.2°.

In certain embodiments, the X-ray powder diffraction pattern exhibits characteristic peaks at 8.5090 ± 0.2°, 11.4131 ± 0.2°, 12.7282 ± 0.2°, 13.0778 ± 0.2°, 13.8888 ± 0.2°, 14.8709 ± 0.2°, 15.8155 ± 0.2°, 16.1708 ± 0.2°,

16.8430 ± 0.2°, 17.0931 ± 0.2°, 18.3337 ± 0.2°, 18.9954 ± 0.2°, 19.6726 ± 0.2°, 19.8849 ± 0.2°, 20.6845 ± 0.2°, 20.8631 ± 0.2°, 21.4220 ± 0.2°, 22.0595 ± 0.2°, 22.2748 ± 0.2°, 23.0060 ± 0.2°, 23.6149 ± 0.2°, 23.8802 ± 0.2°, 24.2450 ± 0.2°, 25.4835 ± 0.2°, 25.7696 ± 0.2°, 26.3492 ± 0.2°, 27.2456 ± 0.2°, 27.5202 ± 0.2°, 27.8593 ± 0.2°, 28.0475 ± 0.2°, 29.5926 ± 0.2°, 30.0442 ± 0.2°, 30.7152 ± 0.2°, 30.9672 ± 0.2°, 31.6849 ± 0.2°, 31.9795 ± 0.2°, 32.4073 ± 0.2°, 33.1395 ± 0.2°, 34.0516 ± 0.2°, 34.8350 ± 0.2°, 35.4536 ± 0.2°, 35.9251 ± 0.2°, 36.5791 ± 0.2°, 37.2047 ± 0.2°, and 38.9204 ± 0.2°.

In certain embodiments, the X-ray powder diffraction pattern expressed in terms of diffraction angle 2θ is shown in Figure 1.

In certain embodiments, the TGA curve of crystalline form I is shown in Figure 2.

In certain embodiments, the DSC curve of crystalline form I is shown in Figure 3.

The present disclosure further provides a method for preparing crystalline form I of the compound represented by formula (I), comprising the steps of: mixing the compound represented by formula (I) with a solvent I, stirring, centrifuging, and drying.

In certain embodiments, the solvent I is selected from ethanol.

The present disclosure further provides a pharmaceutical composition, comprising crystalline form I of the compound represented by formula (I) and optionally a pharmaceutically acceptable carrier, diluent, or excipient.

The present disclosure further provides a method for preparing a pharmaceutical composition, comprising the step of mixing crystalline form I of the compound represented by formula (I) with a pharmaceutically acceptable carrier, diluent, or excipient.

The present disclosure further provides a use of the aforementioned crystalline form I of the compound represented by formula (I) or the aforementioned composition, or a composition prepared by the aforementioned method, in the manufacture of a medicament for preventing and/or treating an HDAC6-mediated or dependent disease or disorder.

In certain embodiments, the HDAC6-mediated or dependent disease or disorder is selected from the group consisting of cancer, neurological diseases, cardiovascular diseases, immune-related diseases, and inflammatory diseases.

The "2θ or 2θ angle" described in the present disclosure refers to the diffraction angle, where θ is the Bragg angle, and the unit is ° or degrees; the error range for the 2θ of each characteristic peak is ±0.2 (including the case where numbers with more than one decimal place are rounded), which can be -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20.

The precipitation methods described in the present disclosure include, but are not limited to, stirring, cooling, evaporation, slurrying, and precipitation.

The "differential scanning calorimetry or DSC" described in the present disclosure refers to measuring the temperature difference and heat flow difference between a sample and a reference during heating or isothermal processes, to characterize all physical and chemical changes related to thermal effects, thereby obtaining phase transition information of the sample.

The drying temperature described in this disclosure is generally 25°C to 100°C, preferably 40°C to 70°C. The drying process can be performed under normal pressure or reduced pressure.

The "pharmaceutical composition" refers to a composition comprising one or more crystalline forms of the compound of formula (I) as described herein, and optionally a pharmaceutically acceptable carrier, diluent, or excipient. The purpose of the pharmaceutical composition is to facilitate administration to an organism, promote absorption of active ingredients, and thereby exert biological activity.

The method of the present disclosure is illustrated below through specific examples. It should be understood that these examples are intended to explain the basic principles, main features, and advantages of the present disclosure, but the scope of the present disclosure is not limited by the following examples. The implementation conditions adopted in the examples may be further adjusted according to specific requirements, and unspecified implementation conditions are generally those used in conventional experiments.

The explanations of the abbreviations used in the present disclosure are as follows:

XRPD: X-ray powder diffraction
DSC: Differential scanning calorimetry
TGA: Thermogravimetric analysis

Instruments and methods used for data collection:

The X-ray powder diffraction patterns described in the examples of the present disclosure were collected on a Panalytical Empyrean X-ray powder diffractometer. The method parameters for the X-ray powder diffraction described in the present disclosure are as follows:

X-ray: Cu, Kα; Kα1 (Å): 1.54060; Kα2 (Å): 1.54443
X-ray tube setting: 45 kV, 40 mA
Scan range (°2θ): 3° to 40°
Scan step size (°2θ): 0.0263
Scan time per step (s): 46.665

The differential scanning calorimetry (DSC) spectra described in the present disclosure were collected on a METTLER DSC 3 differential scanning calorimeter. The method parameters for the differential scanning calorimetry (DSC) described in the present disclosure are as follows:

Method: Linear heating
Sample pan: Aluminum pan, crimped lid
Temperature range: 35-325°C
Heating rate: 10°C/min
Protective gas: Nitrogen

The thermogravimetric analysis (TGA) curve described in the present disclosure were collected on a TA Discovery TGA 5500 thermogravimetric analyzer. The method parameters for the thermogravimetric analysis (TGA) described in the present disclosure are as follows:

Method: Linear heating
Sample pan: Aluminum pan, open
Temperature range: Room temperature to 350°C
Heating rate: 10°C/min
Protective gas: Nitrogen

The high-performance liquid chromatography (HPLC) data described in the present disclosure were collected from a Waters 2489 UV/Vis Detector, with the set parameters as shown in Table 1.

Table 1: HPLC Parameter Table

| Column | Waters Xbridge C18,4.6*50mm, 3.5μm | |
|---|---|---|
| | Gradient | |
| Mobile Phase | A: Water (0.1% phosphoric acid) | B: Acetonitrile |
| 0 min | 80% | 20% |
| 2.5 min | 10% | 90% |
| 3.5 min | 10% | 90% |
| 3.51 min | 80% | 20% |
| 5 min | 80% | 20% |
| Column Temperature (°C) | 30 | |
| Injection Volume (μL) | 5 | |
| UV Wavelength (nm) | 254 | |
| Flow Rate (ml/min) | 2 | |
| Run Time (min) | 5 | |

Method for Stability Testing

Appropriate amounts of crystalline form samples of the compound represented by formula (I) were taken and subjected to stability experiments under conditions of 40°C/75% RH/open/one month. Solid samples separated under different conditions were evaluated for physical stability via XRPD testing for crystalline form and for chemical stability via HPLC testing for purity.

Method for Solubility Testing

An appropriate amount of crystalline form sample of the compound represented by formula (I) was weighed into a vial, an appropriate amount of different media was added, and the mixture was stirred and observed in a 37°C water bath; if the sample dissolved completely, more sample was added until undissolved material remained after rotary mixing; the sample vial was sealed, stirred in a 37°C water bath for 24h, centrifuged, and the supernatant was filtered and tested for solubility by HPLC.

**Example 1: Preparation of the Compound Represented by Formula (I)**

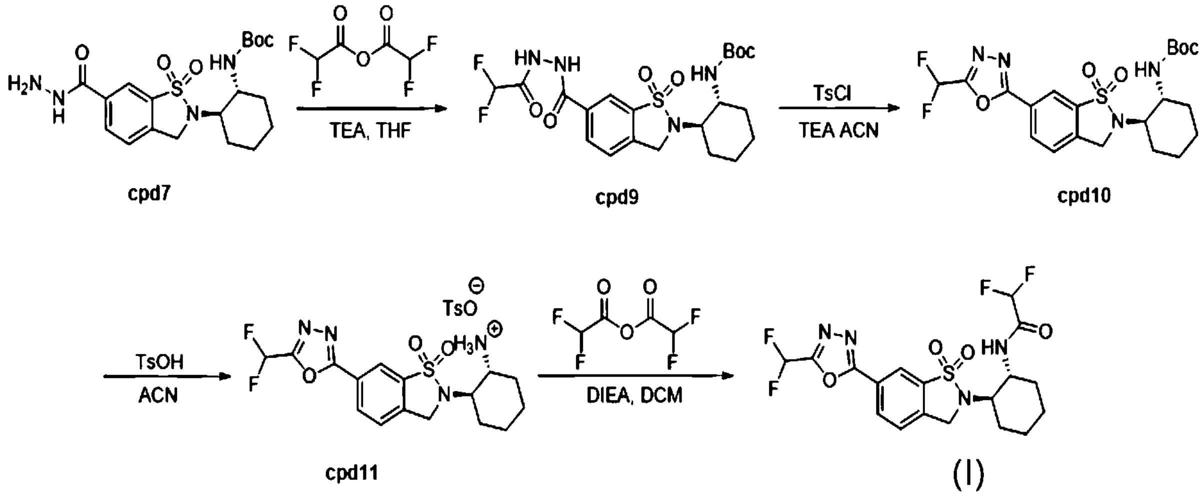


cpd7 cpd9 cpd10

cpd11 (I)

Step 1.

Into a 50L jacketed reactor equipped with a reflux condenser, mechanical stirrer, and thermocouple thermometer were added: 16L of dichloromethane, 2kg of cpd7, 1kg of triethylamine, and 127g of water. Under nitrogen protection and at 0°C, stirring was started. A solution of 1.6kg of difluoroacetic anhydride in dichloromethane was added dropwise to the reaction mixture. After the addition was complete, stirring was continued. Upon completion of the reaction, the solvent was removed under reduced pressure. 5L of methanol was added, and the mixture was stirred until clear. 20L of water was added dropwise, causing solid precipitation. The mixture was filtered by suction, the filter cake was washed with water, and dried to afford cpd9. Material information is shown in Table 2.
ESI m/z $[M-H]^-$ = 501.2。

Table 2 Material Information

| Material Name | Supplier | Quality |
|---|---|---|
| cpd 7 | Shanghai DouChuang Pharmaceutical | HPLC: >98.0% Content: >97.0% |

Step 2.

Into a 50L jacketed reactor equipped with a reflux condenser, mechanical stirrer, and thermocouple thermometer were added: 16L of acetonitrile, 2.25kg of cpd9, and 1.35kg of triethylamine. Under nitrogen protection and at room temperature, stirring was started. A solution of 1.02kg of p-toluenesulfonyl chloride in acetonitrile was added dropwise to the reaction mixture. After the addition was complete, stirring was continued. 22L of ethyl acetate and 22L of water were added, the mixture was stirred, allowed to stand for phase separation, and the aqueous phase was extracted with ethyl acetate. The organic phases were combined, and the solvent was removed under reduced pressure. 7L of isopropanol was added, the mixture was heated to reflux for slurrying, cooled, filtered, washed with an isopropanol-water mixed solvent, and dried to afford cpd10.
ESI m/z $[M-H]^-$ = 483.2。

Step 3.

Into a 50L jacketed reactor equipped with a reflux condenser, mechanical stirrer, and thermocouple thermometer were added: 16L of acetonitrile and 1.6kg of cpd10. At room temperature, stirring was started. A solution of 1.15kg of p-toluenesulfonic acid monohydrate in acetonitrile was added dropwise. After the addition was complete, stirring was continued. Upon completion of the reaction, the mixture was filtered, the filter cake was washed with acetonitrile, and dried to afford cpd11.
ESI m/z $[M+H]^+$ = 385.2。

Step 4.

Into a 50L jacketed reactor equipped with a reflux condenser, mechanical stirrer, thermocouple thermometer, and nitrogen protection were added: 18L of dichloromethane, 1.8kg of cpd11, and 2.1kg of DIPEA. Under nitrogen protection and at -15°C, stirring was started. A solution of 1.13kg of difluoroacetic anhydride in dichloromethane was added dropwise. After the addition was complete, stirring was continued. The temperature was raised to 25°C, and 18L of water was added dropwise. The mixture was stirred and then allowed to stand for phase separation. The organic phase was washed with saturated ammonium chloride and water. The organic phase was collected, part of the solvent was removed under reduced pressure, ethyl acetate was added to replace dichloromethane, isopropanol was added, part of the solvent was removed under reduced pressure, the mixture was heated to reflux for slurrying, cooled, n-heptane was added dropwise, after the addition was complete, stirring was continued, the mixture was filtered by suction, the filter cake was washed with an n-heptane-isopropanol mixed solvent, and dried to afford the compound of formula (I).

ESI m/z $[M+H]^+$ = 463.2.1H NMR (400 MHz, Chloroform-d) δ 8.53 (d, J = 1.5 Hz, 1H), 8.41 (dd, J = 8.1, 1.6 Hz, 1H), 7.67 (d, J = 8.1 Hz, 1H), 6.99 (t, J = 51.6 Hz, 1H), 6.83 (d, J = 9.2 Hz, 1H), 5.74 (t, J = 54.2 Hz, 1H), 4.64 (dd, J = 72.0, 16.0 Hz 2H), 4.07 (qd, J = 10.8, 3.9 Hz, 1H), 3.75 (td, J = 11.4, 3.8 Hz, 1H), 2.20 (ddd, J = 37.9, 14.1, 4.0 Hz, 2H), 2.03 - 1.86 (m, 2H), 1.78 (qd, J = 12.3, 3.4 Hz, 1H), 1.57 - 1.34 (m, 3H)。

**Experimental Example 1: Evaluation of the Inhibitory Activity of the Compound Represented by Formula (I) against HDAC6 Using an Enzyme Activity Assay**

HDAC6 (Abcam), the compound represented by formula (I), and a 20 μM substrate solution (Ac-GAK(Ac)-AMC) were added to a 384-well plate, incubated at 37°C for 30 min, then 1 μM Trypsin and 10 μM TSA were added, incubated at room temperature for 15 min, excited at 360 nm, and the fluorescence emission intensity at 455 nm was detected to calculate the inhibition rate at each concentration. The $IC_{50}$ was obtained by fitting with GraphPad Prism 7.0 software. The experimental results are shown in Table 3.

Table 3: $IC_{50}$ Values of the Compound Represented by Formula (I)

| Compound Number | $IC_{50}$ (nM) |
|---|---|
| **I** | 5.7 |

**Experimental Example 2: Evaluation of the Inhibitory Activity of the Compound Represented by Formula (I) against HDAC1-5 and 7-11 Using an Enzyme Activity Assay**

Different HDAC subtype proteins (purchased from BPS), the compound represented by formula (I), and a substrate solution (purchased from BPS) at concentrations of 2.5-40 μM were added to a 384-well plate, incubated at 37°C for 30 min, then 1 μM Trypsin and 10 μM TSA were added, incubated at room temperature for 15 min, excited at 360 nm, and the fluorescence emission intensity at 455 nm was detected to calculate the inhibition rate at each concentration. The IC50 was obtained by fitting with GraphPad Prism 7.0 software. The experimental results are shown in Table 4 and Table 5.

Table 4: HDAC1 Inhibition Rate/% of the Compound Represented by Formula (I)

| | HDAC1 Inhibition Rate/% | |
|---|---|---|
| Compound Number | 10 μM | 1 μM |
| **I** | -3.91 | -11.89 |

**Table 5:** $IC_{50}$ Values of the Compound Represented by Formula (I)

| Compound Number | $IC_{50}$ (μM) | | | | | | | | | |
|---|---|---|---|---|---|---|---|---|---|---|
| | HDA C 1 | HDA C 2 | HDA C 3 | HDA C 4 | HDA C 5 | HDA C 7 | HDA C 8 | HDA C 9 | HDA C 10 | HDA C 11 |
| **I** | > 50 | > 50 | > 50 | > 50 | > 50 | > 50 | > 50 | > 50 | > 50 | > 50 |

**Experimental Example 3: Test of α-Tubulin Acetylation Level**

A375/HCT116/HCC827 cells in logarithmic growth phase were digested with trypsin cell digestion solution, centrifuged, counted, and seeded into a 96-well plate at a suitable cell density (30,000 cells/well), 100 μL per well, with an appropriate amount of PBS used for water sealing around the wells. On the next day, cells were treated with compounds at different concentrations (starting concentration of 100 μM, 5-fold dilution, with 9 concentration gradients set). After 6h, the medium was aspirated, and cells were washed twice with 100 μL of PBST. Subsequently, cells were fixed with 4% paraformaldehyde and incubated at room temperature for 20 min. The fixative was discarded, and cells were washed with PBST. 150 μL of a dedicated blocking solution (containing 1% Triton-100) was added to each well, and cells were blocked at room temperature for 2h. Excess blocking solution was washed away with PBS. α-Tubulin (DM1A) Mouse mAb and Acetyl-α-Tubulin (Lys40) (D20G3) XP Rabbit mAb were diluted at a ratio of 1:2000 in a blocking solution containing 0.033% Triton-100, added to the 96-well plate, and incubated at 4°C overnight, with a blank control group set simultaneously. On the next day, cells were washed twice with PBST. Secondary antibodies Anti-Mouse IgG (H+L) (DyLight™ 680 Conjugate) and Anti-Rabbit IgG (H+L) (DyLight™ 800 Conjugate) were diluted at a ratio of 1:2000 in a blocking solution containing 0.033% Triton-100, added to the 96-well plate, and incubated at room temperature for 2h. Cells were washed twice with PBST and once with PBS. Fluorescence signals at 700 nm and 800 nm were detected using a Li-COR Odyssey dual-color near-infrared laser imaging system. The experimental results are shown in Table 6.

Table 6: Experimental Results of the α-Tubulin Acetylation Level Test

| Cell | Compound Number | $EC_{50}$ (nM) |
|---|---|---|
| A375 | I | 202.5±114.8 |
| HCT116 | I | 139.9±39.1 |
| HCC827 | I | 287.5±49.3 |

**Experimental Example 4: Stability Test**

Evaluation of the Phase I metabolic stability of the test compound in liver microsomes from CD-1 mice (MLM), Sprague-Dawley rats (RLM), beagle dogs (DLM), cynomolgus monkeys (CLM), and humans (HLM).

Experimental System:

The animal and human liver microsomes used in this test system were purchased from Xenotech, Corning, or other qualified suppliers and stored in a freezer below -60°C before use.

Experimental Overview:

The test and control compounds were incubated with animal and human liver microsomes at 37±1°C for a certain period, with a maximum incubation time of 60 minutes. Samples were taken at specified time points, and the reaction was terminated with acetonitrile or other organic solvents containing an internal standard. After centrifugation, the resulting supernatant was analyzed using liquid chromatography-tandem mass spectrometry (LC-MS/MS).

Experimental Methods:

1. Preparation of Buffer Solution

73.21 g of dipotassium hydrogen phosphate trihydrate and 10.78 g of potassium dihydrogen phosphate were dissolved in 4000 mL of ultrapure water. The pH of the solution was adjusted to 7.40±0.10 using 10% phosphoric acid or 1 M potassium hydroxide, with a final concentration of 100 mM.

2. Preparation of Working Solutions

The test powder was prepared into a stock solution of a certain concentration using dimethyl sulfoxide (DMSO) or other organic solvents, then further diluted with a suitable organic solvent.
Control compounds (testosterone, diclofenac, and propafenone) were prepared into 10 mM stock solutions using DMSO, then further diluted with a suitable organic solvent.

3. Preparation of Liver Microsome Solution

Microsomes from various species were diluted to a 2× working solution using 100 mM potassium phosphate buffer. The final concentration of microsomes in the reaction system was 0.5 mg/mL.

4. Preparation of Reduced Nicotinamide Adenine Dinucleotide Phosphate (NADPH) Regeneration System

Appropriate amounts of nicotinamide adenine dinucleotide phosphate (NADP) and isocitric acid (ISO) powders were weighed, dissolved in magnesium chloride solution, and mixed by vortexing. An appropriate amount of isocitrate dehydrogenase (IDH) was added and mixed gently by inverting. The final concentrations in the reaction system were: 1 mM NADP, 1 mM magnesium chloride, 6 mM ISO, and 1 unit/mL IDH.

5. Preparation of Termination Solution

The termination solution was prepared using acetonitrile or other organic solvents containing an internal standard (tolbutamide or other suitable compound). The prepared termination solution was stored in a refrigerator at 2-8°C.

6. Incubation Process

Incubation was performed in 96-well plates. Eight incubation plates were prepared and named T0, T5, T15, T30, T45, T60, Blank60, and NCF60. The first six plates corresponded to reaction time points of 0, 5, 15, 30, 45, and 60 minutes, respectively. The Blank60 plate did not contain the test article or control compounds, and samples were taken after 60 minutes of incubation. In the NCF60 plate, potassium phosphate buffer was used instead of the NADPH regeneration system solution for incubation for 60 minutes. All conditions were performed in triplicate.
Microsomes were mixed with the test or control compounds. Then, incubation plates Blank60, T5, T15, T30, T45, and T60 (except T0 and NCF60) were placed in a 37°C water bath for pre-incubation for approximately 10 minutes. For incubation plate T0, the termination solution was added first, followed by the NADPH regeneration system working solution. For incubation plate NCF60, 98 μL of potassium phosphate buffer was added to each sample well to initiate the reaction. After pre-incubation of plates Blank60, T5, T15, T30, T45, and T60, 98 μL of the NADPH regeneration system working solution was added to each sample well to initiate the reaction. The reaction temperature was 37±1°C, the final reaction volume was 200 μL, and the reaction system included 0.5 mg/mL microsomes, 1.0 μM substrate, 1 mM NADP, 6 mM ISO, and 1 unit/mL IDH.
At 5, 15, 30, 45, and 60 minutes, cold termination solution containing the internal standard was added to the reaction plates to terminate the reaction.
All terminated reaction plates were shaken and centrifuged at 4°C, 3220 ×g, for 20 minutes. The supernatant was diluted to a certain ratio for LC-MS/MS analysis.

Sample Analysis

Sample analysis was performed using liquid chromatography-tandem mass spectrometry (LC-MS/MS), without calibration curves and quality control samples. Semi-quantitative determination was performed using the ratio of the analyte peak area to the internal standard peak area. The retention times of the analyte and internal standard, chromatogram acquisition, and chromatogram integration were processed using the software Analyst (Sciex, Framingham, Massachusetts, USA).
The CV of the internal standard peak area in each matrix within each analytical batch should be within 20%.

Data Analysis

The in vitro elimination rate constant $k_e$ of the compound was obtained by converting the ratio of the compound to internal standard peak area into the remaining percentage using the formula below:

$$\text{Remaining Percentage (\%)} = \frac{\text{Ratio of compoundto internal standard peak area at any time point}}{\text{Ratio of compound to internal standard peak area at 0 minutes}} \times 100$$

$$C_t = C_0 \bullet e^{-k_e \bullet t}$$

***when***

$$C_t = \frac{1}{2} C_0 ,$$

$$T_{1/2} = \frac{Ln\ 2}{k_e} = \frac{0.693}{k_e}$$

CLint (mic) = 0.693 / T1/2 / Microsomal protein content (microsome concentration mg/mL during incubation)

CLint (liver) = CLint (mic) × Microsomal protein amount in liver (mg/g) × Liver weight to body weight ratio

According to the well-stirred model, hepatic intrinsic clearance and hepatic clearance can be converted using the following formulas.

$$CL(liver) = (CLint(liver) * Qh)/ (CLint(liver) + Qh)$$

The parameters in the formula are shown in Table 7 below.

Table 7: Formula Parameters

| Species | Liver Weight to Body Weight Ratio (g/kg body weight) [1-2] | Hepatic Blood Flow (Qh) (mL/min/kg) [1-2] | Microsomal Protein Content (mg/g liver weight) |
|---|---|---|---|
| Mouse | 88 | 90.0 | 45 |
| Rat | 40 | 55.2 | |
| Dog | 32 | 30.9 | |
| Monkey | 30 | 43.6 | |
| Human | 20 | 20.7 | |

The experimental results are shown in Table 8.

Table 8: Stability Test Results

| Compound Number | | HLM | RLM | MLM | DLM | CLM |
|---|---|---|---|---|---|---|
| I | R2 | 0.7713 | 0.8854 | 0.7684 | 0.5254 | 0.8196 |
| | T1/2 (min) | >145 | >145 | >145 | >145 | >145 |
| | CLint(mic) (μL/min/mg) | <9.6 | <9.6 | <9.6 | <9.6 | <9.6 |
| | CLint(liver) (mL/min/kg) | <8.6 | <17.3 | <38.0 | <13.8 | <13.0 |
| | Remaining Rate (T=60 min) | 86.1% | 76.4% | 84.8% | 85.8% | 88.8% |
| | Remaining Rate (NCF=60 min) | 97.3% | 93.0% | 99.8% | 90.9% | 101.5% |

**Experimental Example 5: Mouse Pharmacokinetic Test**

This experimental example tested the pharmacokinetic behavior of the compound in BALB/c mice after

intravenous (IV) and oral (PO) administration.

The actual body weight of the mice was measured on the day of dosing and the dose volume was calculated. Each group consisted of 3 mice. The compound was tested in two groups: one group received a single intravenous injection, and the other group received a single oral gavage. Whole blood samples were collected via orbital bleeding at specified time points (0.25, 0.5, 1, 2, 4, 8, 24h post-dose). After blood collection, samples were immediately transferred to labeled commercial tubes containing K2-EDTA (0.85-1.15 mg), then centrifuged (3200 x g, 4°C, 10 minutes) to obtain plasma. The plasma was transferred to pre-cooled centrifuge tubes, flash-frozen on dry ice, and stored in an ultra-low temperature freezer at -60°C or lower until LC-MS/MS analysis.

Plasma concentrations were determined using an LC-MS/MS method. The plasma concentration data of the compound of formula (I) were processed using a non-compartmental model with WinNonlin Version 6.3 (Pharsight, Mountain View, CA) pharmacokinetic software. Relevant pharmacokinetic parameters were calculated using the linear log trapezoidal method. The experimental results are shown in Table 9.

Table 9: Mouse Pharmacokinetic Test Experimental Data

| Compou nd Number | | | IV@2mg/kg | PO@10mg/ kg | PO@30mg/ kg | PO@100mg /kg |
|---|---|---|---|---|---|---|
| I | AUC(0-t) | nM*h | 12552.15 | 77690.92 | 182495.53 | 600707.51 |
| | AUC(0-∞) | nM*h | 12552.15 | 77770.78 | 182495.53 | 600707.51 |
| | MRT(0-∞) | h | 2.35 | 4.50 | 3.66 | 4.33 |
| | t1/2z | h | 1.01 | 1.62 | 0.87 | 0.81 |
| | Vz | L/kg | 0.55 | | | |
| | Tmax | h | | 0.50 | 0.67 | 1.33 |
| | CLz | L/h/kg | 0.36 | | | |
| | C0/Cmax | nM | 6749.28 | 13883.48 | 35033.09 | 87871.06 |
| | F | % | | 123.92 | 96.93 | 95.71 |

**Experimental Example 6: Mouse Brain-to-Plasma Drug Concentration Ratio**

The actual body weight of the mice was measured on the day of dosing and the dose volume was calculated. Each group consisted of 9 mice. The compound was administered as a single intravenous (IV) injection or a single oral gavage (PO). Whole blood samples were collected via orbital bleeding at specified time points. After blood collection, samples were immediately transferred to labeled commercial tubes containing K2-EDTA (0.85-1.15 mg), then centrifuged (3200 x g, 4°C, 10 minutes) to obtain plasma. The plasma was transferred to pre-cooled centrifuge tubes, flash-frozen on dry ice, and stored in an ultra-low temperature freezer at -60°C or lower until LC-MS/MS analysis. 20 μL of plasma sample or brain tissue homogenate was added to 80 μL of internal standard solution (50 ng/mL propafenone in acetonitrile). The plate was sealed at 165 °C using a plate sealer, vortexed for 10 min on a microplate shaker (maximum speed), and centrifuged at 4000 rpm for 20 min using a low-speed benchtop centrifuge. 10 μL of the supernatant was transferred and mixed with 90 μL of acetonitrile. After vortex mixing, the plate was sealed at 165 °C, vortexed again, and 1 μL of the supernatant was taken for LC-MS/MS analysis. The plasma concentration data of the compound were processed using a non-compartmental model with WinNonlin Version 6.3 (Pharsight, Mountain View, CA) pharmacokinetic software. Relevant pharmacokinetic parameters were calculated using the linear log trapezoidal method. The experimental results are shown in Table 10.

Data Processing:

Mouse brain-to-plasma drug concentration ratio at the same time point = Drug concentration in brain tissue / Drug concentration in plasma

Table 10: Mouse Brain-to-Plasma Drug Concentration Ratio Experimental Data

| Compound Number | Dose | Time (h) | Brain Drug Concentration (ng/mL) | Plasma Drug Concentration (ng/mL) | Brain/Plasma Drug Concentration Ratio |
|---|---|---|---|---|---|
| I | 2 mg/kg IV | 1 | 268 | 1090 | 0.26 |
| | | 4 | 149 | 595 | 0.25 |
| | 10mg/kg PO | 1 | 882 | 4993 | 0.18 |
| | | 4 | 435 | 2833 | 0.15 |

**Example 2: Preparation of Crystal Form I of the Compound of Formula (I)**

Approximately 20 mg of the sample was weighed into a 5 mL sample vial, and 500 μL of ethanol solvent was added. The vial was placed on a magnetic stirrer (200 rpm) and stirred for 24 hours. After centrifugation at 10,000 r/min for 10 min, the supernatant was decanted. The precipitate was dried in an oven at 40°C. The obtained solid was collected and subjected to XRD testing. The test results are shown in Table 11.

The XRPD pattern is shown in Figure 1, and its characteristic peak positions are shown in Table 11. The TGA pattern is shown in Figure 2, showing a weight loss of 1.35% from 26.8-190°C. The DSC pattern is shown in Figure 3, showing endothermic peaks at 111.59°C and 194.07°C.

Table 11: XRPD Diffraction Peak Data for Crystal Form I

| 2θ[°] | d-spacing [Å] | Relative Intensity [%] |
|---|---|---|
| 8.5090 | 10.39 | 1.86 |
| 11.4131 | 7.75 | 0.60 |
| 12.7282 | 6.96 | 9.94 |
| 13.0778 | 6.77 | 6.56 |
| 13.8888 | 6.38 | 5.63 |
| 14.8709 | 5.96 | 3.91 |
| 15.8155 | 5.60 | 1.52 |
| 16.1708 | 5.48 | 8.30 |
| 16.8430 | 5.26 | 9.73 |
| 17.0931 | 5.19 | 80.30 |
| 18.3337 | 4.84 | 100.00 |
| 18.9954 | 4.67 | 0.83 |
| 19.6726 | 4.51 | 3.31 |
| 19.8849 | 4.47 | 5.66 |
| 20.6845 | 4.29 | 2.09 |
| 20.8631 | 4.26 | 1.44 |
| 21.4220 | 4.15 | 79.74 |
| 22.0595 | 4.03 | 14.24 |
| 22.2748 | 3.99 | 1.94 |
| 23.0060 | 3.87 | 6.70 |
| 23.6149 | 3.77 | 1.21 |
| 23.8802 | 3.73 | 2.34 |
| 24.2450 | 3.67 | 9.07 |
| 25.4835 | 3.50 | 3.26 |
| 25.7696 | 3.46 | 9.17 |

(continued)

| 2θ[°] | d-spacing [Å] | Relative Intensity [%] |
|---|---|---|
| 26.3492 | 3.38 | 10.56 |
| 27.2456 | 3.27 | 1.29 |
| 27.5202 | 3.24 | 4.13 |
| 27.8593 | 3.20 | 2.08 |
| 28.0475 | 3.18 | 1.16 |
| 29.5926 | 3.02 | 6.97 |
| 30.0442 | 2.97 | 5.24 |
| 30.7152 | 2.91 | 1.60 |
| 30.9672 | 2.89 | 0.66 |
| 31.6849 | 2.82 | 2.66 |
| 31.9795 | 2.80 | 0.91 |
| 32.4073 | 2.76 | 1.70 |
| 33.1395 | 2.70 | 0.59 |
| 34.0516 | 2.63 | 0.38 |
| 34.8350 | 2.58 | 1.94 |
| 35.4536 | 2.53 | 1.38 |
| 35.9251 | 2.50 | 0.27 |
| 36.5791 | 2.46 | 0.82 |
| 37.2047 | 2.42 | 1.04 |
| 38.9204 | 2.31 | 1.92 |

**Example 3: Solubility of Crystal Form I of the Compound of Formula (I) in Water**

Approximately 1-2 mg of the crystal form sample of the compound of formula (I) was weighed into a 5 mL vial, and 1 mL of water was added. The vial was stirred and observed in a 37°C water bath. If the sample dissolved completely, more sample was added until undissolved material remained after rotary mixing. The sample vial was sealed and stirred in a 37°C water bath for 24h, then centrifuged. The supernatant was filtered and the solubility was measured by HPLC. The results are shown in Table 12.

Table 12: Solubility of Crystal Forms of the Compound of Formula (I) in Water

| Medium | Time Point (h) | Solubility (μg/mL) |
|---|---|---|
| $H_2O$ | 24h | 49.75 |

**Example 4: Dynamic Solubility of Crystal Form I of the Compound of Formula (I) in Biorelevant Media**

FaSSGF (Fasted State Simulated Gastric Fluid), FeSSGF (Fed State Simulated Gastric Fluid), FaSSIF (Fasted State Simulated Intestinal Fluid), and FeSSIF (Fed State Simulated Intestinal Fluid) are biorelevant media. These media better reflect the impact of the gastrointestinal physiological environment on drug release. Solubility tested in these media is closer to solubility in the human body environment.

Approximately 1-2 mg of the crystal form sample of the compound of formula (I) was weighed into a 5 mL vial, and 1 mL of different media was added. The vial was stirred and observed in a 37°C water bath. If the sample dissolved completely, more sample was added until undissolved material remained after rotary mixing. The sample vial was sealed and stirred in a 37°C water bath for 24h, then centrifuged. The supernatant was filtered and the solubility was measured by HPLC. The results are shown in Table 13.

Table 13: Dynamic Solubility of Crystal Forms in Biorelevant Media

| Medium | Time Point (h) | Solubility (μg/mL) |
|---|---|---|
| FaSSGF | 1h | 34.52 |
| FaSSGF | 24h | 51.85 |
| FaSSIF | 24h | 96.23 |
| FeSSGF | 1h | 45.49 |
| FeSSGF | 24h | 54.97 |
| FeSSIF | 24h | 87.51 |

**Example 5: Stability of Crystal Form I of the Compound of Formula (I)**

Appropriate amounts of crystal form I samples were taken and subjected to a stability experiment under accelerated conditions of 40 °C/75%RH/open/1 month. Physical stability was evaluated by XRPD testing. The XRPD patterns before and after storage are shown in Figure 4, and chemical stability is shown in Table 14.

Table 14: Stability of crystal form I

| | | | T0 | 40 °C/75%RH/open/1 month |
|---|---|---|---|---|
| | Name | RT (min) | % Area | % Area |
| 1 | RRT~ 0.756 | 7.92 | 0.02 | 0.02 |
| 2 | RRT~ 0.816 | 8.536 | / | 0.03 |
| 3 | RRT~ 0.819 | 8.578 | 0.03 | 0.04 |
| 4 | RRT~ 0.857 | 8.973 | 0.07 | 0.07 |
| 5 | RRT~ 0.934 | 9.783 | 0.04 | 0.03 |
| 6 | Compound of formula (I) | 10.471 | 99.51 | 99.51 |
| 7 | RRT~ 1.029 | 10.77 | 0.03 | 0.02 |
| 8 | RRT~ 1.039 | 10.873 | / | / |
| 9 | RRT~ 1.107 | 11.589 | 0.17 | 0.17 |
| 10 | RRT~ 1.129 | 11.819 | 0.03 | 0.02 |
| 11 | RRT~ 1.252 | 13.106 | / | / |
| 12 | RRT~ 1.257 | 13.157 | 0.1 | 0.1 |

The XRPD pattern results show that the characteristic peaks of crystal form I remained unchanged under the accelerated conditions of 40 °C/75%RH/open/1 month, indicating good stability.

It should be understood that the above examples are exemplary and are not intended to encompass all possible implementations covered by the claims. Various modifications and changes can be made to the above examples without departing from the scope of the present disclosure. Similarly, the various technical features of the above examples can be arbitrarily combined to form other examples of the present disclosure that may not have been explicitly described. Therefore, the above examples merely express several implementations of the present disclosure and do not limit the scope of protection of the patent of the present disclosure.

## Claims

**1.** A crystal form I of a fused ring compound, the structural formula of the fused ring compound being:

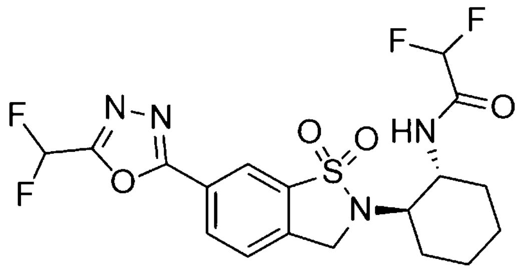

(I),

wherein the X-ray powder diffraction pattern, expressed in terms of diffraction angle 2θ, exhibits characteristic peaks at 17.0931 ± 0.2°, 18.3337 ± 0.2°, 21.4220 ± 0.2°, 22.0595 ± 0.2°, and 26.3492 ± 0.2°; preferably, it further exhibits characteristic peaks at 12.7282 ± 0.2°, 16.1708 ± 0.2°, 16.8430 ± 0.2°, 17.0931 ± 0.2°, 18.3337 ± 0.2°, 21.4220 ± 0.2°, 22.0595 ± 0.2°, 24.2450 ± 0.2°, 25.7696 ± 0.2°, and 26.3492 ± 0.2°; more preferably, it further exhibits characteristic peaks at 12.7282 ± 0.2°, 13.0778 ± 0.2°, 13.8888 ± 0.2°, 16.1708 ± 0.2°, 16.8430 ± 0.2°, 17.0931 ± 0.2°, 18.3337 ± 0.2°, 19.8849 ± 0.2°, 21.4220 ± 0.2°, 22.0595 ± 0.2°, 23.0060 ± 0.2°, 24.2450 ± 0.2°, 25.7696 ± 0.2°, 26.3492 ± 0.2°, and 29.5926 ± 0.2°; most preferably, it further exhibits characteristic peaks at 12.7282 ± 0.2°, 13.0778 ± 0.2°, 13.8888 ± 0.2°, 14.8709 ± 0.2°, 16.1708 ± 0.2°, 16.8430 ± 0.2°, 17.0931 ± 0.2°, 18.3337 ± 0.2°, 19.6726 ± 0.2°, 19.8849 ± 0.2°, 21.4220 ± 0.2°, 22.0595 ± 0.2°, 23.0060 ± 0.2°, 24.2450 ± 0.2°, 25.4835 ± 0.2°, 25.7696 ± 0.2°, 26.3492 ± 0.2°, 27.5202 ± 0.2°, 29.5926 ± 0.2°, and 30.0442 ± 0.2°.

2. The crystal form I according to claim 1, wherein the X-ray powder diffraction pattern exhibits characteristic peaks at 8.5090±0.2°, 11.4131±0.2°, 12.7282±0.2°, 13.0778±0.2°, 13.8888±0.2°, 14.8709±0.2°, 15.8155±0.2°, 16.1708 ±0.2°, 16.8430±0.2°, 17.0931±0.2°, 18.3337±0.2°, 18.9954±0.2°, 19.6726±0.2°, 19.8849±0.2°, 20.6845±0.2°, 20.8631±0.2°, 21.4220±0.2°, 22.0595±0.2°, 22.2748±0.2°, 23.0060±0.2°, 23.6149±0.2°, 23.8802±0.2°, 24.2450±0.2°, 25.4835±0.2°, 25.7696±0.2°, 26.3492±0.2°, 27.2456±0.2°, 27.5202±0.2°, 27.8593±0.2°, 28.0475±0.2°, 29.5926±0.2°, 30.0442±0.2°, 30.7152±0.2°, 30.9672±0.2°, 31.6849±0.2°, 31.9795±0.2°, 32.4073±0.2°, 33.1395±0.2°, 34.0516±0.2°, 34.8350±0.2°, 35.4536±0.2°, 35.9251±0.2°, 36.5791±0.2°, 37.2047±0.2°, and 38.9204±0.2°.

3. The crystal form I according to claim 2, wherein the X-ray powder diffraction pattern is as shown in Figure 1.

4. The crystal form I according to claim 3, wherein the TGA pattern of crystal form I is as shown in Figure 2; preferably, the DSC pattern of crystal form I is as shown in Figure 3.

5. A method for preparing the crystal form I of the compound of formula (I) according to any one of claims 1-4, wherein comprising the steps of: mixing the compound of formula (I) with a solvent I, stirring, centrifuging, and drying.

6. The method for preparing crystal form I according to claim 5, wherein the solvent I is selected from ethanol.

7. A pharmaceutical composition prepared from the crystal form I of the compound of formula (I) according to any one of claims 1-4.

8. A pharmaceutical composition, comprising the crystal form I of the compound of formula (I) according to any one of claims 1-4 and optionally a pharmaceutically acceptable carrier, diluent, or excipient.

9. A method for preparing a pharmaceutical composition, comprising the step of mixing the crystal form I of the compound of formula (I) according to any one of claims 1-4 with a pharmaceutically acceptable carrier, diluent, or excipient.

10. Use of the crystal form I of the compound of formula (I) according to any one of claims 1-4, or the composition according to claims 7 or 8, or the composition prepared by the method according to claim 9, in the manufacture of a medicament for preventing and/or treating an HDAC6-mediated or -dependent disease or disorder;
preferably, the HDAC6-mediated or -dependent disease or disorder is selected from cancer, neurological diseases, cardiovascular diseases, immune-related diseases, and inflammatory diseases.

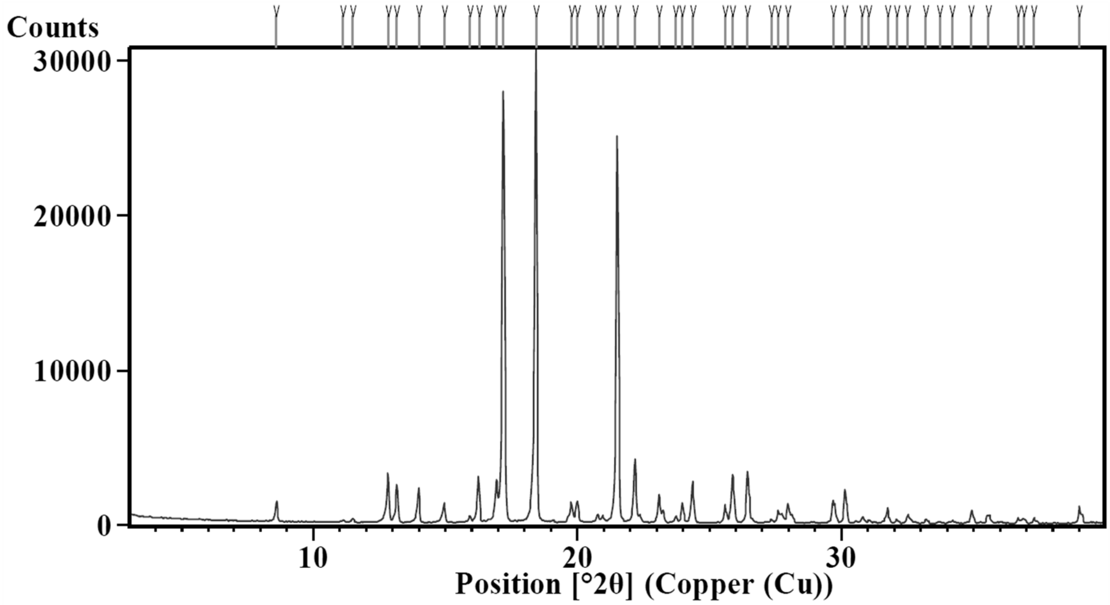


Figure 1

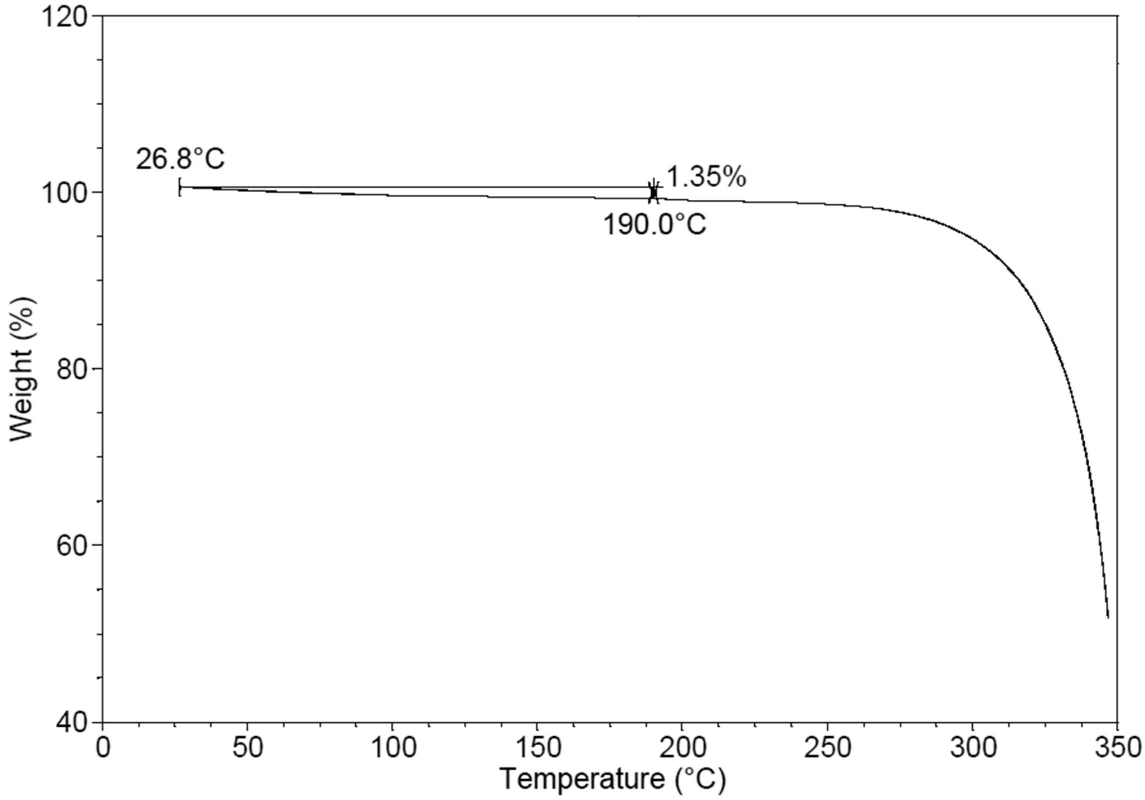


Figure 2

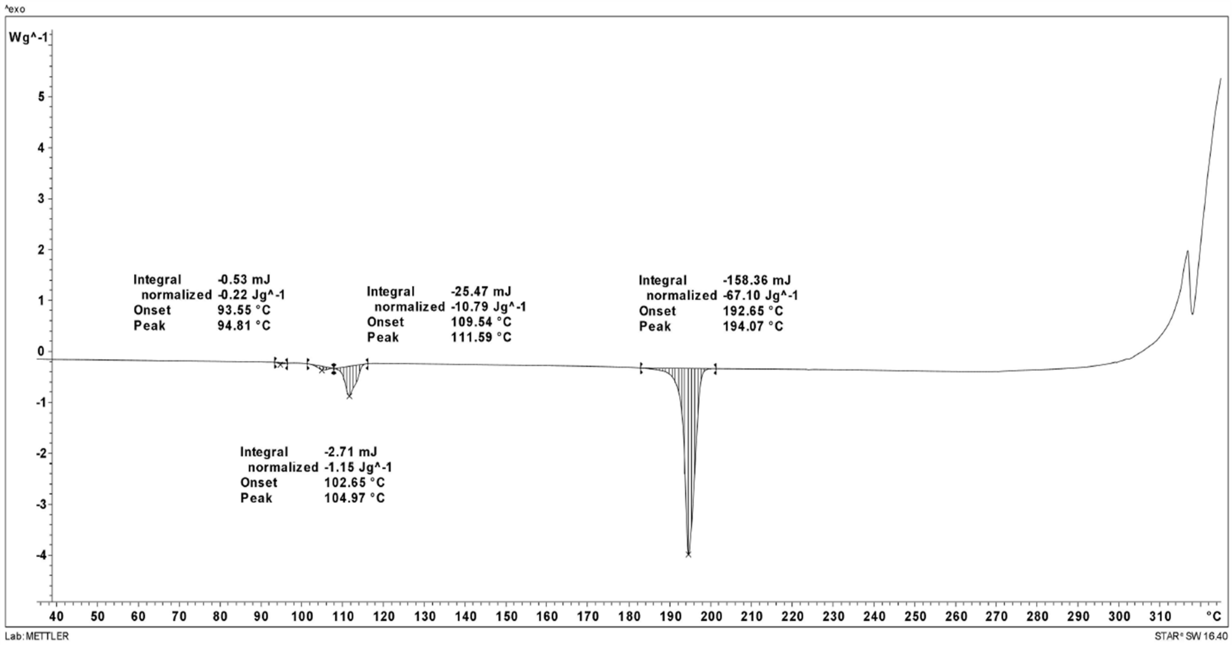


**Figure 3**

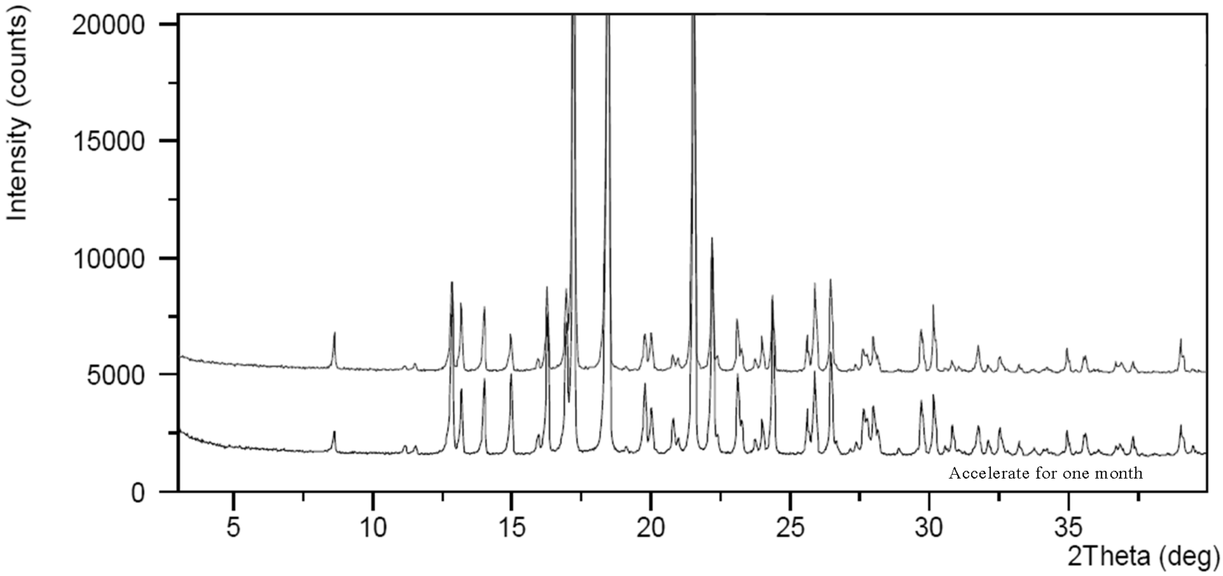


**Figure 4**

## INTERNATIONAL SEARCH REPORT

International application No.
**PCT/CN2024/121715**

**A. CLASSIFICATION OF SUBJECT MATTER**

C07D 417/04(2006.01)i; A61K 31/545(2006.01)i; A61K 31/5415(2006.01)i; A61P 35/00(2006.01)i; A61P 25/28(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, STN (REGISTRY, CAPLUS): 噁二唑, 磺酰胺, 组蛋白去乙酰化酶抑制剂, 中枢神经系统, 癌症, HDAC, oxadiazol, sulfonamide, histone deacetylase, central nervous system disease, cancer, tumor, STN结构式检索, STN structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 111032651 A (TAKEDA PHARMACEUTICAL CO., LTD.) 17 April 2020 (2020-04-17)<br>description, page 1, paragraph 1, and pages 198-264, tables 1-1 to 1-67, and claims 1-22 | 1-10 |
| A | US 2022098180 A1 (TAKEDA PHARMACEUTICAL CO., LTD.) 31 March 2022 (2022-03-31)<br>description, page 3, paragraphs 20-22, and pages 73-86, table 3, and claims 1-16 | 1-10 |
| A | CN 114846012 A (TAKEDA PHARMACEUTICAL CO., LTD.) 02 August 2022 (2022-08-02)<br>description, page 1, paragraph 1, and pages 43-64, tables 1-1 to 1-22, and claims 1-15 | 1-10 |
| PX | WO 2023198172 A1 (SUZHOU GENHOUSE BIO CO., LTD.) 19 October 2023 (2023-10-19)<br>embodiment 2 | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 December 2024** | **06 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/121715**

| Patent document cited in search report | Publication date (day/month/year) | Patent family member(s) | Publication date (day/month/year) |
|---|---|---|---|
| CN 111032651 A | 17 April 2020 | US 2019135799 A1 | 09 May 2019 |
| | | US 10435399 B2 | 08 October 2019 |
| | | JP 2020529393 A | 08 October 2020 |
| | | JP 7135007 B2 | 12 September 2022 |
| | | TW 201910328 A | 16 March 2019 |
| | | WO 2019027054 A1 | 07 February 2019 |
| | | CA 3071825 A1 | 07 February 2019 |
| | | EP 3661931 A1 | 10 June 2020 |
| US 2022098180 A1 | 31 March 2022 | JPWO 2020158762 A1 | 02 December 2021 |
| | | JP 7470058 B2 | 17 April 2024 |
| | | US 12084436 B2 | 10 September 2024 |
| | | EP 3919055 A1 | 08 December 2021 |
| | | EP 3919055 A4 | 09 November 2022 |
| | | WO 2020158762 A1 | 06 August 2020 |
| CN 114846012 A | 02 August 2022 | AU 2020353984 A1 | 21 April 2022 |
| | | AU 2020353984 A2 | 04 August 2022 |
| | | ECSP 22026524 A | 31 May 2022 |
| | | JP 2022552444 A | 15 December 2022 |
| | | US 2021094944 A1 | 01 April 2021 |
| | | US 11453661 B2 | 27 September 2022 |
| | | BR 112022005595 A2 | 19 July 2022 |
| | | KR 20220070465 A | 31 May 2022 |
| | | TW 202126643 A | 16 July 2021 |
| | | EP 4034536 A1 | 03 August 2022 |
| | | CL 2022000763 A1 | 20 January 2023 |
| | | WO 2021060567 A1 | 01 April 2021 |
| | | ZA 202204666 B | 29 November 2023 |
| | | IL 291413 A | 01 May 2022 |
| | | IL 291413 B1 | 01 November 2024 |
| | | CO 2022005059 A2 | 08 July 2022 |
| | | PE 20220711 A1 | 04 May 2022 |
| | | AR 120040 A1 | 26 January 2022 |
| | | US 2023183227 A1 | 15 June 2023 |
| | | US 11958845 B2 | 16 April 2024 |
| | | CA 3156060 A1 | 01 April 2021 |
| | | MX 2022003490 A | 25 April 2022 |
| | | US 2024343721 A1 | 17 October 2024 |
| WO 2023198172 A1 | 19 October 2023 | TW 202400147 A | 01 January 2024 |
| | | AU 2023254010 A1 | 21 November 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311277270